(19) Europäisches Patentamt

European Patent Office

Office européen des brevets

(11) Publication number: **0 211 506**
**B1**

(12) **EUROPEAN PATENT SPECIFICATION**

(45) Date of publication of patent specification: 04.04.90

(21) Application number: 86304935.9

(22) Date of filing: 25.06.86

(51) Int. Cl.⁵: **C 12 P 19/04,** A 23 L 1/054,
C 12 R 1/05

(54) **Heteropolysaccharide and its Production and use.**

(30) Priority: 28.06.85 US 750806

(43) Date of publication of application:
25.02.87 Bulletin 87/09

(45) Publication of the grant of the patent:
04.04.90 Bulletin 90/14

(84) Designated Contracting States:
AT BE CH DE FR GB IT LI LU NL SE

(56) References cited:
EP-A-0 064 354
EP-A-0 079 038
US-A-4 269 939

CHEMICAL ABSTRACTS, vol. 78, no. 9, 5th
March 1973, page 371, abstract no. 56454d,
Columbus, Ohio, US; & JP-A-72 25 390 (JAPAN.
BUREAU OF INDUSTRIAL TECHNOLOGY) 20-10-
1972

CHEMICAL ABSTRACTS, vol. 96, no. 1, January
1982, page 460, abstract no. 4951r, Columbus,
Ohio. US; & JP-A-81 109 593 (AGENCY OF
INDUSTRIAL SCIENCES AND TECHNOLOGY
TOKUYAMA SODA CO. LTD.) 31-08-1981

(73) Proprietor: MERCK & CO. INC.
126, East Lincoln Avenue P.O. Box 2000
Rahway New Jersey 07065-0900 (US)

(72) Inventor: Peik, Jerry A.
11291 Tribuna Avenue
San Diego California 92131 (US)
Inventor: Steenbergen, Suzanna M.
3105 Victoria Drive P O Box 636
Alpine California 92001 (US)
Inventor: Veeder, George T.
4494 Pocahontas Avenue
San Diego California 92117 (US)

(74) Representative: Crampton, Keith John Allen
et al
D YOUNG & CO 10 Staple Inn
London WC1V 7RD (GB)

Courier Press, Leamington Spa, England.

# EP 0 211 506 B1

**Description**

This invention relates to microbial polysaccharides. It is known that a common feature of certain microorganisms is the production of exocellular heteropolysaccharides. Heteropolysaccharides are generally linear carbohydrate polymers of high molecular weight containing two or more kinds of monosaccharides that form a repeating unit that is polymerized.

The usefulness of most heteropolysaccharides is based on their ability to alter the viscosity and rheology of aqueous solutions. In addition, heteropolysaccharides have related secondary functions, such as emulsification, suspension, stabilization, flocculation, lubrication and film-formation.

Heteropolysaccharides are widely used in food, well-drilling, agriculture and a wide variety of other industrial applications. Commercial demand for these water-soluble gums has greatly increased over the last few decades. Furthermore, new industrial techniques create a need for heteropolysaccharides with new physical properties. Consequently, the need for heteropolysaccharides with different functionality ranges, coupled with commercial demand, has clearly indicated the necessity for the development of new heteropolysaccharides with new and different physical properties.

The present invention is based on the discovery that a novel heteropolysaccharide, herein called "Heteropolysaccharide S-184", which is composed principally of carbohydrate, from 12% to 16% protein and from 3.0% to 4.5% (calculated as acetic acid) acyl groups, the carbohydrate portion containing from 7% to 16% uronic acid and the neutral sugars mannose, glucose and galactose in the approximate molar ratios of 1:3:5, and the amount of pyruvic acid being less than 0.1% is produced by the action of a new *Alcaligenes* strain on a selected carbon source. This novel compound, which constitutes one embodiment of the present invention, is prepared by aerobic fermentation of a suitable aqueous nutrient medium with an unnamed *Alcaligenes* strain. A deposited under the Budapest Treaty of a biologically pure culture of this organism was made with the American Type Culture Collection, Rockville, Maryland, on 19 June, 1985, under Accession No. ATCC 53160. The novel microorganism and the production of Heteropolysaccharide S-184 by growing it in an aqueous nutrient medium by aerobic fermentation of an assimilable carbon source and recovering the S-184, constitute other embodiments of the present invention. Heteropolysaccharide S-184 has desirable properties in aqueous systems and is especially useful in foods.

The novel organism of the present invention was isolated from a water sample collected from Pauma Creek on Polomar Mountain in San Diego, California. The organism was picked as a gummy colony from a YM agar plate after 4 days of incubation at 30°C. The isolate was then pure cultured on nutrient agar.

A flask seed was started from a nutrient agar culture of the isolate. This seed was then used to inoculate another flask containing a nutrient medium having hydrolysed starch as the carbon source. After incubation, this flask was noted to contain a viscous beer and upon addition of isopropyl alcohol a fibrous material was precipitated. Another flask seed was started and used to determine the effect of various nutrient media on gum production and to determine the best growth media and fermentation conditions for this microorganism.

Heteropolysaccharide S-184 is produced during the aerobic fermentation of suitable aqueous nutrient media under controlled conditions by inoculation with a culture of the organism ATCC 53160. The media contain sources of assimilable carbon and nitrogen, and inorganic salts.

In general, carbohydrates (for example, glucose, fructose, maltose and xylose) can be used either alone or in combination as sources of assimilable carbon in the nutrient medium. The exact quantity of the carbohydrate source or sources required in the medium depends in part upon the other ingredients of the medium but usually lies in the range 2% to 5% by weight of the medium. These carbon sources may be used individually or combined in the medium.

Generally, many proteinaceous materials may be used as nitrogen sources for the fermentation process. Suitable nitrogen sources include, for example, yeast hydrolysates, primary yeast, soybean meal, cottonseed flour, hydrolysates of casein, cornsteep liquor, distiller's solubles and tomato paste. The sources of nitrogen, either alone or in combination, are used in amounts preferably ranging from 0.05% to 0.2% by weight of the aqueous medium.

Among the nutrient inorganic salts that can be incorporated in the culture media are the customary salts capable of yielding ions such as those of sodium, potassium, ammonium, calcium, phosphate, sulphate, chloride and carbonate. Also included are trace metals such as cobalt, manganese, iron and magnesium.

The nutrient media described herein are illustrative of and do not limit the wide variety of suitable media.

As an alternative medium, S-184 may be grown under low calcium ion conditions, i.e., in deionized water or some other aqueous system substantially free of calcium ions (i.e., less than 4 ppm $Ca^{++}$ per 1% gum in the final fermentor broth).

The fermentation is carried out at temperatures ranging from 25°C to 35°C, optimum results being obtained at from 28°C to 32°C. The pH of the nutrient media for growing the ATCC 53160 culture and producing the heteropolysaccharide S-184 can vary from 6 to 8.

Although S-184 is produced by both surface and submerged culture, it is preferred to carry out the fermentation in the submerged state.

A small-scale fermentation is conveniently carried out by inoculating a suitable nutrient medium with

2

# EP 0 211 506 B1

the culture and, after transfer to a production medium, permitting the fermentation to proceed at a constant temperature of about 30°C on a shaker for several days.

The fermentation is initiated in a sterilized flask of medium via one or more stages of seed development. The nutrient medium for the seed stage may be any suitable combination of carbon and nitrogen sources; however, the preferred carbon source is glucose or hydrolyzed starch. The seed flask is shaken in a constant temperature chamber at about 30°C for 1—2 days, or until growth is satisfactory, and some of the resulting growth is used to inoculate either a second stage seed or the production medium. Intermediate stage seed flasks, when used, are developed in essentially the same manner; that is, part of the contents of the flask from the last seed stage are used to inoculate the production medium. The inoculated flasks are shaken at a constant temperature for several days, and at the end of the incubation period the contents of the flasks are recovered by precipitation with a suitable alcohol such as isopropanol.

For large scale work, it is preferable to conduct the fermentation in suitable tanks provided with an agitator and a means for aerating the fermentation medium. According to this method, the nutrient medium is make up in the tank and sterilized by heating at temperatures of up to about 121°C. Upon cooling, the sterilized medium is inoculated with a previously grown seed of the producing culture, and the fermentation is permitted to proceed for a period of time, for example from 2 to 4 days, while agitating and/or aerating the nutrient medium and maintaining the temperature at about 30°C. This method of producing S-184 is particularly suited for the preparation of large quantities.

In the detailed description below, the words "Difco", "Dowex", "Polysorbate" and "Promosoy" are Registered Trade Marks, and parts are by weight unless otherwise indicated.

Heteropolysaccharide S-184

The heteropolysaccharide produced by ATCC 53160 is composed principally of carbohydrate, from 12% to 16% protein and from 3.0% to 4.5% (calculated as acetic acid) acyl groups, the carbohydrate portion containing from about 7% to 16% uronic acid and the neutral sugars mannose, glucose and galactose in the approximate molar ratios of 1:3:5, the amount of pyruvic acid being less than 0.1%.

A summary of the compositional analysis of heteropolysaccharide S-184 is provided in Table 1, below.

### TABLE 1
#### Composition analysis of heteropolysaccharide S-184

| Analyses | Sample | | | | |
|---|---|---|---|---|---|
| | BD-1842 | BD-1827 | BD-707 | BD-2117 | BD-2118 |
| % protein (N×6.25%) | 15.8 | 12.3 | 12.6 | 11.8 | 12.4 |
| % Uronic acid (mol. wt. 176, decarboxylation) | 15.8 | 12.3 | 10.76 | 9.0 | 7.1 |
| % Acyl (as acetic acid) | (1) 3.9 | (1) 3.1 | (2) 4.5 | (2) 3.4 | (2) 3.6 |
| % Pyruvate | <0.1 | <0.1 | 0 | 0 | 0 |
| Neutral sugars: (% molar ratios) mannose | (3) 12 | (3) 12 | | (4) 19.4 (17.9—23.8) | (5) 9.4 (9.3—9.8) |
| Glucose | 35.6 | 35 | | 30.1 (27.6—31.9) | 29.2 (28.7—29.5) |
| Galactose | 52.4 | 52.5 | | 50.5 (48.5—52.1) | 61.4 (61.1—62.0) |

Notes (1) to (5) represent the analytical procedures described *infra*.

(1) The acetyl contents were determined by treating a 0.2% aqueous solution of S-184 gum with an alkaline, hydroxylamine reagent followed by treatment with an acidic ferric chloride reagent and colorimetric analysis [See S. Hestrin (1949), *J. Biol. Chem. 180*, 249—261]. Acetyl choline chloride was used as the standard.

(2) The acyl content of from 3.1% to 4.5% (calculated as acetic acid) was determined by an enzymatic assay after hydrolysis of O-acetyl linkages using dilute alkali.

(3) The neutral sugars of S-184 were determined by various techniques. The first method involved hydrolysing 50 mg of S-184 in $1M$ $H_2SO_4$ at 100°C for 4 hours. After cooling, 0.5 ml of 3 mg/ml xylose was added as an internal standard. Samples were neutralized by adding 3 ml of saturated $Ba(OH)_2$, then two drops of Congo Red and $Ba(OH)_2$ until the color changed to red. After centrifuging (20 minutes at 3000 RPM) the supernatants of all samples were evaporated. Dry samples were dissolved in 0.1 ml of hydroxylamine hydrochloride (40 mg/ml) in dry pyridine and heated at 90°C for 45 minutes. After cooling, 0.1 ml acetic anhydride was added and the sample again heated at 90°C for 45 minutes. The sugars were separated by gas-liquid chromatography of their aldononitrile acetate derivatives and were identified and quantified by comparison with authentic standards [J. K. Baird, M. J. Holroyde, and D. C. Ellwood (1973) Carbohydr. Res. 27, 464—467].

(4) Samples were pretreated by dissolution in 72% $H_2SO_4$ and held for 1 hour at 0°C prior to dilution and hydrolysis by the first method of neutral sugar analysis described above; results are mean values and ranges obtained for BD-707, BD-2117 and BD-2118.

(5) The neutral sugars of these samples were also characterized by a second method which involves dissolving approximately 2 mg of S-184 in $0.5M$ trifluoroacetic acid (2 ml). The sample was kept at 100°C overnight, concentrated to dryness and dissolved in water (2 ml). Sodium borohydride (25 mg) was added and after 2 hours the solution was treated with Dowex 50 ($H^+$) after which the pH dropped to 3.5. After filtration, the solution was concentrated and codistilled with methanol ($3 \times 5$ ml). The residue was dissolved in a mixture of acetic anhydride (1 ml) and pyridine (1 ml), kept at 100°C for 1 hour and concentrated. After codistillation with toluene ($3 \times 5$ ml), the residue was dissolved in methylene chloride and analysed by gas-liquid chromatography. Results are mean values and ranges obtained for BD-707, BD-2117 and BD-2118.

The % uronic acid of the polysaccharide was determined by decarboxylation with 17% hydrochloric acid, followed by trapping the liberated carbon dioxide in standard sodium hydroxide and back tritration [B. L. Browning (1967) Methods of Wood Chemistry 2, 632—633].

The absence of pyruvate was determined by adding 1 ml of a 2 mg/ml solution of S-184 to a culture tube, and adding 1 ml of 0.2N HCl, and heating at 100°C for 4 hours. A 0.5 ml sample of hydrolysate was added to 0.1 ml of reduced nicotinamide adenine dinucleotide (NADH) and 2.4 ml of triethanolamine solution. The absorbance change was measured on a spectrophotometer and pyruvate measured. [Duckworth and Yaphe Chem. & Ind. 1970) p. 747]. No significant pyruvate was detected.

Nitrogen analysis was performed by Kjeldahl digestion and was determined to be between approximately 1.9% and 2.5% by weight corresponding to 11.8%—15.8% protein equivalents.

Methylation analysis was performed on partially purified samples of S-184 after dialysis and freeze drying. The samples were methylated according to the procedures outlined in Sandford & Conrad, (1966) Biochem. 5, 1508—1507. The O-methyl ether derivatives of the sugars as their aditol acetates were separated by gas chromatography and identified by computer matching with authentic standards. The major methylated sugars identified are shown in Table 2, below.

TABLE 2
Methylated sugar residues of S-184

| Identified sugar residue | Linkage |
| --- | --- |
| 2, 3, 6 $Me_3$ Hexitol (Galactose) | 1—4 |
| 2, 3, 6 $Me_3$ Hexitol (Mannose) | 1—4 |
| 2, 3, 6 $Me_3$ Hexitol (Glucose) | 1—4 |

It is to be understood that, although the methods of analysis of the heteropolysaccharide described herein were the actual methods used in arriving at the composition described above, other methods of analysis are available. Each methods of analysis should result in the same characterization of the heteropolysaccharide; however, slightly different quantitative results may be reported.

Heteropolysaccharide S-184 has been found to have oustanding properties in aqueous solution, especially in having high viscosity at very low concentrations, good surface activity, good protein compatibility and excellent stabilization/emulsification properties. Because of this, it is useful as a thickening, suspending, emulsifying, stabilizing, lubricating, film-forming, or binding agent and as a replacement for gum arabic in many applications. S-184 has utility in various industrial and food applications where surface activity and protein compatibility are desirable. In particular it has uses in the following applications or products: adhesives, wall joint cements, water-retentive grouts and mortars, spackling compounds, can sealants, boiler compounds, latex creaming, welding-rod fluxes, braising pastes, ceramic glazes and extrusions, cleaners and polishers, toys, emulsions (latex, asphalt, silicone), silver recovery, seed coatings, spray control for pesticides or herbicides, emulsifiable concentrates and flowable pesticides and herbicides, tobacco binders, water based inks, lithographic fountain solutions, leather finishes, hydromulching and hydro-seeding, textile printing and finishing, wet-end paper additives, wet-end paper retention and formation aids, anti-slick compounds, mold-release agents, liquid resins, slurry and packaged explosives, petroleum and water-well drilling muds, petroleum workover and completion fluids, petroleum stimulation fluids, cosmetics, pharmaceutical suspensions and emulsions.

This gum also has utility in food systems such as jellies and other high-sugar systems, beverages including citric acid based drinks, dairy products including ice cream and yogurt, salad dressings, dry mixes, icings, and glazes, syrups, puddings, farinaceous foods, canned and retorted foods and bakery fillings.

A particular valuable utility is in the area of food applications, especially salad dressing, beverage and dairy products. Heteropolysaccharide S-184 has been found to be particularly useful in foods because of its surface activity, protein compatibility and stabilization/emulsification properties.

A salad dressing was prepared by slurrying heteropolysaccharide S-184 in two to five times its weight of oil, adding the S-184/oil slurry to water and hydrating under vigorous agitation, blending all other dry ingredients and adding them to the water/slurry blend, adding tomato paste and mixing for 3 minutes, adding oleoresin paprika and mixing for 3 minutes, adding oil slowly and mixing for 3 minutes, adding vinegar and mixing for 3 minutes, homogenizing using a Colloid Mill and bottling.

S-184 was added as an emulsion stabilizer to the dressing at amounts between about 0.10% and 0.40% by weight. Viscosity, pH, storage stability and texture of the dressing were determined and are shown in Table 3, below.

TABLE 3
Heteropolysaccharide S-184 in salad dressing

| Use level % | Viscosity "mPa·s" (24 hours) | pH | Stability (days) | | Texture, flow property |
|---|---|---|---|---|---|
| | | | 49°C (120°F) | Room temp. | |
| 0.10 | 1000 | 3.6 | 7 | 83 | Smooth thin flow |
| 0.15 | 1450 | 3.6 | 31 | 61 | |
| 0.20 | 1650 | 3.6 | 26 | 71 | Smooth, creamy |
| 0.25 | 2700 | 3.6 | 33 | Over 365 | Stable, acceptable |
| 0.40 | 4700 | 3.6 | Not tested | | Semi-gelled, unacceptable |

Heteropolysaccharide S-184 has a particular profile of solution properties that is a distinctive characteristic of this polysaccharide and which enables it to be distinguished over other heteropolysaccharide. S-184 has the following profile of properties:

1. Viscosity and shear
A. Brookfield

| | | Syn. Tap. H$_2$O* mPa·s | Syn. Tap H$_2$O* +0.1% KCl mPa·s |
|---|---|---|---|
| 1. | 1.0% @ 60 rpm | 1080 | 1030 |
| | @ 6 rpm Spindle No. 3 | 6000 | |
| 2. | 0.1% (UL adapter @ 6 rpm) | 7 | 7 |
| 3. | 0.5% Wells-Brookfield @ 9.6 sec$^{-1}$ | 330 | 320 |
| 4. | 1.0% DI H$_2$O @ 60 rpm Spindle No. 3 | 1460 | |

*Deionized water containing 1000 ppm NaCl and 147 ppm CaCl$_2$·2H$_2$O.

B. Shear (Wells-Brookfield in mPa · s)
    1. $\eta$ @ 1.92 sec$^{-1}$ 4100
    2. $\eta$ @ 9.6 sec$^{-1}$ 1270
    3. $\eta$ @ 76.8 sec$^{-1}$ 290
    4. $\eta$ @ 384 sec$^{-1}$ greater than 60 (initial)
    5. $\eta$ @ 384 sec$^{-1}$ greater than 60 (5 min)
    6. $\eta$ @ 9.6 sec$^{-1}$ 1180

C. 4.5°C (40°F) storage (Brookfield)
    1320 mPa · s @ 60 rpm with Spindle No. 3; no gelation.

2. Salt and dye compatibility

| | | |
|---|---|---|
| A. | Salt | |
| | 1. CaCl$_2$ (saturated) | Compatible |
| | 2. Amm. polyphosphate | Precipitate |
| | 3. 60% NH$_4$NO$_3$ | Compatible |
| | 4. 1% Al$_2$(SO$_4$)$_3$ · 18H$_2$O | Compatible |
| | 5. 1% CaCl$_2$ · 2H$_2$O | Compatible |
| | 6. 1% KCl | Compatible |
| B. | Dyes | |
| | 1. Milling Green | Compatible |
| | 2. Methylene Blue | Compatible |

Description of the strain
    · Heteropolysaccharide S-184 may be prepared by fermentation of a suitable nutrient medium with an unnamed *Alcaligenes* species. A deposit under the Budapest Treaty of a biologically pure culture of the microorganism employed in making this heteropolysaccharide was made with the American Type Culture Collection, Rockville, Maryland, on June 19, 1985 under Accession No. ATCC 53160.

A. Characteristics of colony morphology
    On nutrient agar isolate ATCC 53160 forms round colonies with opaque centers and translucent edges. The pigmentation is white to grey. Colonies are 1—3 mm after 72 hours at 30°C.

B. Characteristics of cell morphology
    The cells are gram-negative, long rods (0.8—2.3 μm) with vacuoles present. The flagella arrangement was mixed as polar, lateral and peritrichous forms were observed. The organism was observed to produce a pinkish pigment under some growth conditions.

C. Physiological and biochemical characteristics
    The physiological and biochemical test results are given in Table 4, below. The organism is cytochrome oxidase positive, catalase positive and oxidative in metabolism. The results were positive for citrate utilization and production of H$_2$S. It has the ability to hydrolyze starch, gelatin, casein and Polysorbate 80, USP (a surface active agent available from Difco). It will grow well at 30°C and 37°C but was not tested at higher temperatures. It can tolerate 1.5% NaCl but not 3% and will grow in the pH range of 6—10.
    Its pattern of acid production from carbohydrates is shown below:

Acid production
_____

L-Arabinose
Fructose
Galactose
Maltose (weak)
Mannitol (weak)
Mannose

No acid production
_____

Adonitol
Dulcitol
Ethanol
D-Glucose
D-Xylose
Inositol
Inulin
Lactose
Melibiose
α-Methylglucoside
Raffinose
Rhamnose
Salicin
Sorbitol
Sucrose
Trehalose
D-Xylose

ATCC 53160 was able to utilize the following 46 substrates as sole carbon and energy sources:

D-Xylose            L-Malate
L-Arabinose         DL-β-Hydroxybutyrate
D-Glucose           DL-Lactate
D-Mannose           DL-Glycerate
D-Galactose         Citrate
D-Fructose          α-Ketoglutarate
Maltose             Pyruvate
Cellobiose          Laevulinate
Inulin              Mannitol
Gluconate           Glycerol
Saccharate          D-α-Alanine
Mucate              β-Alanine
Acetate             L-Threonine
Propionate          L-Leucine
Butyrate            DL-Isoleucine
Caproate            L-Aspartate
Heptanoate          L-Glutamate
Caprylate           L-Lysine
Pelargonate         DL-Ornithine
Caprate             L-Histine
Malonate            L-Proline
Succinate           L-Tyrosine
Fumarate            L-Phenylalanine

7

TABLE 4
Physiological and biochemical test results
of strain ATCC 53160

| | |
|---|---|
| Cytochrome oxidase | + |
| Catalase | + |
| OF tests | Oxidative |
| Anaerobic growth | − |
| TSI agar: Slant | NC |
| Indole | − |
| Methyl Red | − |
| Voges-Proskauer | − |
| Simmons' citrate | + |
| Nitrite reduction | − |
| Nitrate reduction | − |
| Litmus milk | NC |
| Arginine dehydrolase | − |
| Lysine decarboxylase | − |
| Ornithine decarboxylase | − |
| Phenylalanine deaminase | NG |
| Ammonia from peptone | − |
| Urease | − |
| H₂S (from peptone, cystine, and sulfite medium) | + |
| 3-Ketolactose | − |
| Congo Red absorption | − |
| Phosphatase | − |
| Haemolysis (sheep blood) | NT |
| Egg yolk reaction | ± |
| Starch hydrolysis | + |
| Gelatin hydrolysis | + |
| Casein hydrolysis | + |
| Esculin hydrolysis | − |
| Polysorbate 80 hydrolysis | + |
| Growth at various temperatures: | |
| 4°C | − |
| 30°C | + |
| 37°C | + |
| Growth at various NaCl concentrations: | |
| 1.5% (W/V) | + |
| 3.0% (W/V) | − |
| 6.5% (W/V) | − |
| 7.5% (W/V) | − |
| 10.0% (W/V) | − |
| Growth at various pH values: | |
| 4 | − |
| 6 | + |
| 8 | + |
| 10 | + |
| 11 | + |
| 12 | − |

NG=No growth
NC=No change
NT=Not tested
+=positive
−=negative

Since the organism was strictly aerobic, gram negative, and showed mixed flagellation, it was classified as belonging to the genus *Alcaligenes*, according to the 8th edition of Bergey's Manual of Determinative Bacteriology (1974).

The genus *Alcaligenes* was redefined in the latest Bergey's Manual (Bergey's Manual of Systematic Microbiology, Vol. 1, 1st Edition, 1984) to exclude all pigmented organisms. Isolate ATCC 53160 fits within this newly defined genus but does not belong to either of the two species listed which are *Alcaligenes*

8

*faecalis, Alcaligenes denitrificans* subspecies *denitrificans* and *Alcaligenes denitrificans* subspecies *xylosoxidans*. The strain ATCC 53160 is thus representative of a new species of *Alcaligenes*.

The following Examples are illustrative.

Example 1

A YM flask seed was started from a 48-hour nutrient agar culture placed on a gyrotary shaker at 30°C. Approximately 24 hours later, this seed was used to inoculate a flask containing $E_1$ medium with 3% hydrolyzed starch as the carbon source. This flask was also placed on a gyrotary shaker at 30°C. Approximately 72 hours later, this flask was noted to have viscous beer, and upon addition of 2 volumes of 99% isopropyl alcohol, a fibrous precipitate was noted.

$E_1$ medium contains 5 g of dipotassium phosphate, 0.1 g of magnesium sulfate, 0.9 g of ammonium nitrate, 0.5 g of Promosoy 100 (an enzymatic digest of soybean meal sold by Central Soya Chemurgy Division), 30 g of dextrose and 1 L of tap water. The pH and the $E_1$ medium is about 7.6—7.8.

Another YM seed flask was prepared in the above fashion and used at 24 hours to inoculate 4 flasks containing various media and these flasks were incubated at 30°C on a gyrotary shaker for about 72 hours at which time the pH, viscosity, gum yield and product viscosity were measured. The results are shown in Table 5 below.

TABLE 5

Effect of media on gum production

| Medium | Carbon source | pH | Beer vis. (mPa · s) | % Gum yield | DI water prod. vis. (mPa · s) 1%/0.1% | 1% KCl prod. vis. (mPa · s) 1%/0.1% |
|---|---|---|---|---|---|---|
| $E_1$ | 3% glucose | 6.8 | 7600 | 1.702 | 1900/15 | 1100/7 |
| $E_1$ (—$NH_4NO_3$ + 0.19% $NaNO_3$) | 3% glucose | 7.6 | 7400 | 1.840 | ND | ND |
| $E_1$ (containing 0.2% Promosoy) | 3% glucose | 6.9 | 7200 | 1.762 | ND | ND |
| $E_1$ + HoLe salts[2] | 3% glucose | 6.9 | 2900 | 1.266 | ND | ND |

[1]ND: Not determined.

[2]HoLe salts are prepared by adding the following ingredients to one liter of deionized or distilled water:

| | |
|---|---|
| $H_3BO_3$ | 285 mg |
| $MnCl_2 \cdot 4H_2O$ | 1800 mg |
| $FeSO_4$ | 1360 mg |
| $CuCl_2$ | 26.9 mg |
| $ZnCl_2$ | 20.8 mg |
| $CoCl_2$ | 40.4 mg |
| $Mg_2MoO_4 \cdot 2H_2O$ | 25.2 mg |
| Sodium tartrate | 1770 mg |

This stock solution is added to media at 1 ml/L.

As seen from the above results, the best growth medium is $E_1$ with 3% glucose.

Example 2

A fermentation procedure for producing large quantities of heteropolysaccharide S-184 is provided.

A 500 ml Erlenmeyer flask containing 100 ml of YM broth (Difco) was inoculated with a loopful of ATCC 53160 cells from a 48-hour nutrient agar plate. The flask was incubated for 24 hours at 30°C on a gyrotary shaker set at 400 rpm. A 1% inoculum was then made into two 500 ml Erlenmeyer flasks containing 100 ml each of seed medium. The seed medium contained:

| | |
|---|---|
| Glucose | 3% |
| $K_2HPO_4$ | 0.5% |
| $NH_4NO_3$ | 0.09% |
| $MgSo_4 \cdot 7H_2O$ | 0.01% |
| Promosoy 100 | 0.05% |

9

The medium was prepared in tap water. These flasks were incubated at 30°C on a gyrotary shaker at 400 rpm for 24 hours at which point they were used to inoculate a 5 L fermentor vessel containing 3000 ml (final volume) of the same medium. The fermentation was controlled at 30°C and the aeration rate at 1 L/minute. The agitation was started at 400 rpm and increased thereafter to ensure good mixing. At 24 hours approximately 2.5 L of this seed were used to inoculate a 30 L fermentor containing 20 L (final volume) of the following medium:

| | |
|---|---|
| Glucose | 3.0% |
| $K_2HPO_4$ | 0.05% |
| $NH_4NO_3$ | 0.09% |
| $MgSO_4 \cdot 7H_2O$ | 0.01% |
| Promosoy 100 | 0.05% |
| $Fe^{++}$ | 1 ppm |
| Sag 5691 | 0.005% |
| (a defoaming agent supplied by Union Carbide) | |

The temperature was maintained at 30°C and the aeration rate at 5 L/minute until 22 hours into the fermentation where it was adjusted to 10 L/minute. It remained at that rate for the remainder of the fermentation. The pH was controlled at greater than 6.3 by the addition of 40% KOH as needed using an automatic pH control system. The agitation was initially set at 300 rpm and was increased to 400 rpm at 22 hours and to 700 rpm at 45 hours. It remained at 700 rpm for the remainder of the fermentation. The results of this fermentation are given in Table 6 below.

## TABLE 6

| Age | pH | Beer viscosity (mPa · s) | Gum yield (g/100 ml) | Residual carbon source (%) |
|---|---|---|---|---|
| 0 hours | 7.0 | ND | ND | 3.0 |
| 22 hours | 6.3 | 620 | ND | ND |
| 45 hours | 6.9 | 4200 | 1.26 | 0.41 |
| 68 hours | 7.2 | 5100 | 1.37 | less than 0.1 |

ND=No data

The fermentation liquor was heated to approximately 75°C for 15 minutes and then cooled to approximately 30°C. The fermentation liquor was added to three volumes of 99% isopropanol. The polysaccharide precipitated as a fibrous material which was easily recovered using a sieve. The fibers were dried in a forced air tray drier at 60°C (140°F) for 2.5 hours before being milled to a powder.

Example 3

A 500-ml Erlenmeyer flask containing 100 ml of YM broth (Difco) was inoculated with a loopful of ATCC 53160 cells from a 48-hour nutrient agar plate. The flask was incubated for 24 hours at 30°C on a gyrotary shaker set at 400 rpm. A 1% inoculum was then made into five 500-ml Erlenmeyer flasks containing 100 ml each of seed medium. The seed medium contained:

| | |
|---|---|
| Glucose | 3% |
| $K_2HPO_4$ | 0.5% |
| $NH_4NO_3$ | 0.09% |
| $MgSO_4 \cdot 7H_2O$ | 0.01% |
| Promosoy 100 | 0.05% |
| $Fe^{++}$ | 1 ppm |
| Tap water | |

These flasks were incubated at 30°C on a gyrotary shaker at 400/rpm for 24 hours at which point they were used to inoculate a 14 L fermentor vessel containing 10 L (final volume) of the final medium. The fermentation was controlled at 30°C and the aeration rate at 3 L/minute. The agitation was started at 400 rpm and increased thereafter to ensure good mixing. The final medium consisted of:

| Glucose | 3.0% |
|---|---|
| $K_2HPO_4$ | 0.05% |
| $NH_4NO_3$ | 0.09% |
| $MgSO_4 \cdot 7H_2O$ | 0.01% |
| Promosoy 100 | 0.05% |
| $Fe^{++}$ | 1 ppm |

The pH was controlled at >6.5 by the addition of 25% KOH as needed using an automatic pH control system. The results of this fermentation are given in Table 7 below:

### TABLE 7

| Age | pH | Beer viscosity (mPa · s) | Gum yield gm/100 ml) | Residual carbon source (%) |
|---|---|---|---|---|
| 0 hours | 7.3 | ND | ND | 3.15 |
| 21 hours | 7.05 | 20 | 0.65% | 1.59 |
| 45 hours | 6.9 | 3450 | 1.22% | 0.48 |
| 78 hours | 7.1 | 2550 | 1.50% | less than 0.10 |

ND=No data

The fermentation liquor was heated to approximately 75°C for 15 minutes and then cooled to approximately 30°C. The fermentation liquor was added to three volumes of 99% isopropanol. The polysaccharide precipitated as a vibrous material which was easily recovered using a sieve. The fibers were dried in a forced air tray drier at 60°C (140°F) for 2.5 hours before being milled to a powder.

## Claims

1. Heteropolysaccharide S-184, which is obtained by aerobic fermentation of an aqueous nutrient medium using an *Alcaligenes* microorganism, ATCC 53160, and which comprises principally carbohydrate, from 12% to 16% protein and from 3.0% to 4.5% (calculated as acetic acid) acyl groups, the carbohydrate portion containing from about 7% to 16% uronic acid, and the neutral sugars mannose, glucose and galactose in the approximate molar ratios of 1:3:5, the amount of pyruvic acid being less than 0.1%.

2. A biologically pure culture of an *Alcaligenes* microorganism, ATCC 53160.

3. A culture comprising the *Alcaligenes* microorganism, ATCC 53160, the said culture being capable of producing heteropolysaccharide S-184 in recoverable amounts by aerobic fermentation.

4. A process for producing heteropolysaccharide S-184 which comprises growing the *Alcaligenes* microorganism, ATCC 53160, in an aqueous nutrient medium by aerobic fermentation of an assimilable carbon source and recovering the heteropolysaccharide S-184.

5. A process as claimed in Claim 4, in which the assimilable carbon source is a carbohydrate.

6. A process as claimed in Claim 5, in which the carbohydrate is glucose.

7. A process as claimed in any one of Claims 4 to 6 in which the nutrient medium is substantially free of calcium ions.

## Patentansprüche

1. Heteropolysaccharid S-184, welches durch aerobe Fermentation eines wässrigen Nährmediums unter Verwendung eines Alcaligenes-Mickroorganismus, ATCC 53160, erhältlich ist und welches hauptsächlich Kohlehydrat, von 12% bis 16% Protein und von 3,0% bis 4,5% Acylgruppen (berechnet als Essigsäure) beinhaltet, wobei der Kohlehydratanteil von etwa 7% bis 16% Uronsäure und die neutralen Zucker Mannose, Glucose und Galactose in den ungefähren Molverhältnissen von 1:3:5 enthält, wobei die Menge an Brenztraubensäure weniger als 0,1% beträgt.

2. Biologisch reine Kultur eines Alcaligenes-Mikroorganismus, ATCC 53160.

3. Kultur, umfassend den Alcaligenes-Mikroorganismus, ATCC 53160, welche Kultur fähig ist, durch aerobe Fermentation Heteropolysaccharid S-184 in gewinnbaren Mengen zu produzieren.

4. Verfahren zur Herstellung von Heteropolysaccharid S-184, welches das Züchten des Alcaligenes-Mikroorganismus, ATCC 53160, in einem wässerigen Nährmedium durch aerobe Fermentation einer assimilierbaren Kohlenstoffquelle und das Gewinnen des Heteropolysaccharids S-184 umfaßt.

5. Verfahren nach Anspruch 4, worin die assimilierbare Kohlenstoffquelle ein Kohlehydrat ist.

6. Verfahren nach Anspruch 5, worin das Kohlehydrat Glucose ist.

11

7. Verfahren nach einem der Ansprüche 4 bis 6, worin das Nährmedium im wesentlichen von Calciumionen frei ist.

**Revendications**

1. Hétéropolysaccharide S-184 que l'on peut obtenir par fermentation aérobie d'un milieu nutritif aqueux en utilisant un microorganisme *Alcaligenes* ATCC 53160 et qui comprend principalement des glucides, de 12% à 16% de protéines et de 3,0% à 4,5% (calculé en acide acétique) de groupes acyles, la portion glucidique contenant d'environ 7% à 16% d'acide uronique et les sucres neutres mannose, glucose et galactose dans les rapports molaires approximatifs de 1/3/5, la quantité d'acide pyruvique étant inférieure à 0,1%.

2. Culture biologiquement pure d'un microorganisme *Alcaligenes* ATCC 53160.

3. Culture comprenant le microorganisme *Alcaligenes* ATCC 53160, ladite culture étant capable de produire l'hétéropolysaccharide S-184 en des quantités récupérables par fermentation aérobie.

4. Procédé pour produire l'hétéropolysaccharide S-184, qui comprend la culture du microorganisme *Alcaligenes* ATCC 53160 dans un milieu nutritif aqueux par fermentation aérobie d'une source de carbone assimilable et la récupération de l'hétéropolysaccharide S-184.

5. Procédé selon la revendication 4, dans lequel la source de carbone assimilable est un glucide.

6. Procédé selon la revendication 5, dans lequel le glucide est le glucose.

7. Procédé selon l'une quelconque des revendications 4 à 6, dans lequel le milieu nutritif est essentiellement dépourvu d'ions calcium.